(19) Europäisches Patentamt / European Patent Office / Office européen des brevets

(11) EP 4 620 947 A1

(12) **EUROPEAN PATENT APPLICATION**
published in accordance with Art. 153(4) EPC

(43) Date of publication:
**24.09.2025 Bulletin 2025/39**

(21) Application number: **22965505.5**

(22) Date of filing: **17.11.2022**

(51) International Patent Classification (IPC):
***C07C 263/10*** *(2006.01)* ***C07C 265/14*** *(2006.01)*
***C08G 18/76*** *(2006.01)* ***C08G 18/75*** *(2006.01)*
***C08G 18/38*** *(2006.01)* ***C08G 18/72*** *(2006.01)*
***G02B 1/00*** *(2006.01)* ***G02B 1/04*** *(2006.01)*

(86) International application number:
**PCT/CN2022/132417**

(87) International publication number:
**WO 2024/103321 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Wanhua Chemical Group Co., Ltd.**
**Yantai, Shandong 264006 (CN)**

(72) Inventors:
- **ZHU, Fulin**
  **Yantai, Shandong 264000 (CN)**
- **JIANG, Tengfei**
  **Yantai, Shandong 264000 (CN)**
- **SHANG, Yonghua**
  **Yantai, Shandong 264000 (CN)**
- **LI, Jianfeng**
  **Yantai, Shandong 264000 (CN)**
- **REN, Yizhen**
  **Yantai, Shandong 264000 (CN)**
- **GUO, Yaoyun**
  **Yantai, Shandong 264000 (CN)**
- **WANG, Pengfei**
  **Yantai, Shandong 264000 (CN)**
- **YIN, Li**
  **Yantai, Shandong 264000 (CN)**
- **LI, Yuan**
  **Yantai, Shandong 264000 (CN)**

(74) Representative: **Boult Wade Tennant LLP**
**Salisbury Square House**
**8 Salisbury Square**
**London EC4Y 8AP (GB)**

# (54) ISOCYANATE COMPOSITION, MODIFIED ISOCYANATE, POLYURETHANE RESIN AND OPTICAL MATERIAL

(57) Provided are an isocyanate composition, a modified isocyanate, a polyurethane resin, and optical materials, and an effective factor of the isocyanate composition is 3.90 to 5.70. By the design and control of effective factor, the isocyanate composition has excellent reactivity and can be used for the preparation of high-performance resins such as polyurethane and polythiourethane without causing undesirable phenomena such as gelation during polymerization. **In** addition, the isocyanate composition can effectively improve the yellowing resistance, stability and optical performance of polyurethane, polythiourethane and other products, so that the obtained polyurethane and polythiourethane will not exhibit white turbidity and opacity phenomena. The optical material prepared with the isocyanate composition has remarkably reduced yellowness index and remarkably reduced optical deformation occurrence rate, thereby effectively improving the optical properties of the optical material.

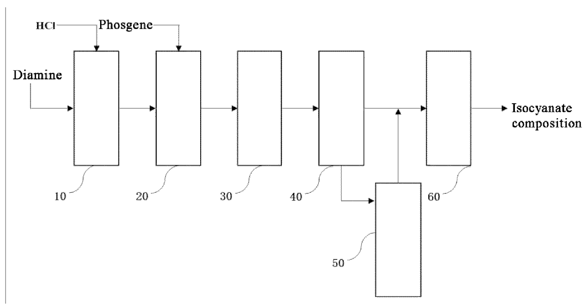


FIG. 1

EP 4 620 947 A1

## Description

### TECHNICAL FIELD

Embodiments of the present application relate to the technical field of isocyanate, for example, an isocyanate composition, a modified isocyanate, a polyurethane resin, and an optical material.

### BACKGROUND

Compared with inorganic optical materials such as glass, optical resin materials have the advantages of being light-weight, not easy to break, not easy to fracture, and easy to dye, and are widely used in fields such as eyeglass lenses, mobile phone lenses, light-fixtures, lenses, prisms, and electrical equipment. At present, known optical resin materials include polycarbonate (PC), polymethyl methacrylate (acrylic, PMMA), polyallyl diglycol carbonate (PADC) resin, polythiourethane and the like, among which polythiourethane has excellent comprehensive performance in terms of transparency, refractive index, impact resistance, dyeability, and processability, and is one of the most promising optical materials.

Polythiourethane is an important branch of polyurethane materials, and is obtained by polymerization of polythiol compounds and polyiso (thio) cyanate compounds. For example, CN101155848A discloses a polythiourethane-based polymerizable composition including the following components: (A) an alicyclic isocyanate compound, (B) an aliphatic isocyanate compound, (C) at least one of a polythiol compound having one or more sulfur bonds in one molecule or a polythiol compound having one or more polysulfur bonds in one molecule, (D) at least one of a polyhydroxy compound having two or more hydroxyl groups in one molecule, and/or a hydroxyl-thio compound having one or more hydroxyl groups and one or more thiol groups. The polythiourethane obtained from the polymerizable composition has good dyeability and strength. CN106414538A discloses a composition for preparing transparent polythiourethane bodies, including a polyisocyanate component, a thiol component and an optional auxiliary agent and additive; where the polyisocyanate component is prepared by gas-phase phosgenation of aliphatic polyamine, cycloaliphatic polyamine, aromatic polyamine or araliphatic polyamine and includes at least one nitrile compound in an amount of at least 0.005 wt% based on the polyisocyanate component, the nitrile compound being derived from the same polyamine as the polyisocyanate; polythiourethane lens prepared by the aforementioned composition has good transparency. CN108084386A discloses a polythiourethane resin composition for optical materials, which is obtained by heating and curing a composition containing an organic zirconium-bismuth alloy complex, an isocyanate-based compound and a thiol-based compound, where the organic zirconium-bismuth alloy complex is used as a catalyst and can improve the yield of lens products in the industrialization process without losing resin properties such as transparency, mechanical properties and heat resistance for plastic lens applications.

The polythiourethane disclosed in the related art mainly focuses on transparency, mechanical properties, dyeability, heat resistance and the like of the material. However, during polymerization of the polythiourethane material, the phenomena of resin turbid and opacity often occur, and the obtained polythiourethane exhibits problems such as yellowing and optical deformation, resulting in disqualification of products such as eyeglasses and lenses, and seriously limiting the wide application of the polythiourethane in optical components such as eyeglasses and lenses.

Therefore, developing polythiourethane with more excellent performance, especially reducing the yellowness index and optical deformation occurrence rate of polythiourethane, is an urgent problem to be solved in the art.

### SUMMARY

The following is a summary of the subjects detailed in the present application. This summary is not intended to limit the scope of the claims.

Embodiments of the present application provide an isocyanate composition, modified isocyanate, polyurethane resin, and optical materials. By designing and controlling an effective factor of the isocyanate composition, the yellowing resistance and stability of polyurethane resin and polythiourethane can be effectively improved, a yellowness index and an optical deformation occurrence rate of the optical materials can be remarkably reduced, and excellent optical properties can be endowed.

In a first aspect, embodiments of the present application provide an isocyanate composition having an effective factor of 3.90 to 5.70.

A calculation formula of the effective factor is shown by Formula I:

$$E = -\lg\left(A - \frac{B \times M_{Cl}}{M_B}\right)$$ Formula I.

In formula I, E is an effective factor.

In formula I, A is a mass content of chlorine in the isocyanate composition.

In formula I, B is a mass content of chloroisocyanate in the isocyanate composition.

In formula I, $M_{Cl}$ is relative atomic mass of chlorine.

In formula I, $M_B$ is relative molecular mass of the chloroisocyanate.

The effective factor E of the isocyanate composition provided by the present application is 3.90 to 5.70, for example, it may be 4.00, 4.10, 4.30, 4.50, 4.70, 4.90, 5.00, 5.10, 5.20, 5.30, 5.40, 5.50 or 5.60, or, specific point value therebetween. Considering limited space and brevity, the present application will not list exhaustively the specific point values encompassed by the ranges.

In the present application, the isocyanate composition includes a combination of isocyanate and chlorine-containing substance, and is therefore defined as a "composition". The chlorine-containing substance includes chloroisocyanate and substance corresponding to an effective factor. According to the present application, by designing and controlling the effective factor, the isocyanate composition may include specific type of chlorine-containing substances with specific content, so that the isocyanate composition has excellent reactivity and can be used for preparation of resins such as high-performance polyurethane and polythiourethane without causing undesirable phenomena such as gelation during polymerization. In addition, the isocyanate composition can effectively improve the yellowing resistance, stability and optical performance of resin products such as polyurethane and polythiourethane, so that the obtained polyurethane and polythiourethane will not exhibit turbid and opacity phenomena, and have low yellowness index and low optical deformation occurrence rate as optical materials, thereby effectively improving the optical quality of the optical materials. If the effective factor is too high, the isocyanate composition used for the preparation of polyurethane or polythiourethane may cause a gelation phenomenon in the polymerization reaction; if the effective factor is too low, it will affect the yellowing resistance and optical properties of polyurethane and polythiourethane, leading to a higher yellowing index of optical materials and an increased probability of optical deformation.

In the present application, in the formula I for calculating the effective factor, A is the mass content of chlorine in the isocyanate composition, preferably determined by X-ray fluorescence (XRF) testing.

Preferably, in the formula I for calculating the effective factor, B is the mass content of chloroisocyanate in the isocyanate composition measured by chromatography-mass spectrometry, preferably by gas chromatography-mass spectrometry (GCMS).

In the research of the present application, it is found that the method for characterizing the chlorine content in isocyanate known in the related art cannot accurately control the performance of isocyanate, so that the quality of polyurethane/polythiourethane, especially the yellowing resistance and optical performance, cannot be effectively controlled. Specifically, the test method for total chlorine content in Standard GB/T 12009.1-1989 is oxygen flask combustion method, in which all chlorine (also including bromine) in isocyanate is converted into inorganic chlorine (also including bromine) and then titrated with silver nitrate to characterize all chlorine content in isocyanate, including bromine content in isocyanate. Standard GB/T 12009.2-2016 measures hydrolysable chlorine, specifically chlorine released after isocyanate reacts with alcohol and water, which is the one with higher activity in isocyanate, in which bromine with higher activity is also included and partial monochloroisocyanate can also be hydrolyzed. Chlorine (also including some bromine) content determined by GB/T 12009.1-1989 or GB/T 12009.2-2016 cannot accurately indicate the component information of isocyanate, nor can it effectively control the performance of isocyanate and polyurethane/polyurethane.

As a preferred technical solution of the present application, in the calculation of the effective factor E, A is the total chlorine content (excluding bromine) obtained by XRF testing, B is the chloroisocyanate content obtained by chromatography-mass spectrometry testing. The A value and the B value are obtained by accurate qualitative and quantitative analysis methods, so that the effective factor E accurately characterizes the polychlorinated compounds and partial hydrolyzed chlorine in the isocyanate composition (excluding the hydrolyzed chlorine of monochloroisocyanate), and corresponds to a finer and clearer chlorine content that plays a key role in the activity of isocyanate and the performance of polyurethane, polythiourethane and other products. As a result, the performance control of the isocyanate composition can be realized, and the performance of polyurethane and polythiourethane prepared thereby can be further improved. Especially, the optical properties (yellowing index, optical deformation) of optical materials can be significantly enhanced.

Preferably, the isocyanate is diisocyanate, and further preferably includes any one of phenylenedimethylene diisocyanate (XDI), bis(isocyanatemethyl)cyclohexane (H6XDI), and bis(isocyanatemethyl)norborane (NBDI), or a combination of at least two thereof.

In the present application, unless otherwise specified, the exemplified isocyanate includes all isomers thereof.

For example, the phenylenedimethylene diisocyanate (XDI) is

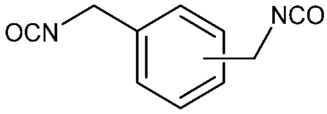

,

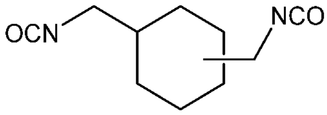

,

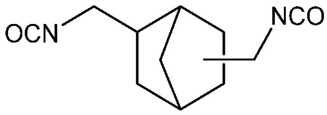

.

the bis(isocyanatemethyl)cyclohexane (H6XDI) is the bis(isocyanatemethyl)norborane (NBDI) is

Preferably, the mass percentage of isocyanate in the isocyanate composition is greater than or equal to 97%, for example, it is 97.5%, 98%, 98.5%, 99%, 99.1%, 99.2%, 99.3%, 99.4%, 99.5%, 99.6%, 99.7%, 99.8%, 99.9%, 99.92%, 99.95%, 99.98%, 99.99%, etc.

Preferably, the mass content of chlorine in the isocyanate composition (A-value) is 2 ppm to 1000 ppm, for example, it may be 5 ppm, 10 ppm, 50 ppm, 100 ppm, 150 ppm, 200 ppm, 250 ppm, 300 ppm, 350 ppm, 400 ppm, 450 ppm, 500 ppm, 550 ppm, 600 ppm, 650 ppm, 700 ppm, 750 ppm, 800 ppm, 850 ppm, 900 ppm or 950 ppm, or, specific point values therebetween. Considering limited space and brevity, the present application will not list exhaustively the specific point values encompassed by the ranges. More preferably, A-value is 15 ppm to 500 ppm.

In the present application, "ppm" means parts per million, and 1 ppm represents one part per million. Hereinafter, same expressions have the same meaning.

Preferably, the substance corresponding to the effective factor includes any one of the following compounds or a combination of at least two thereof:

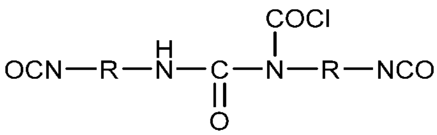

,

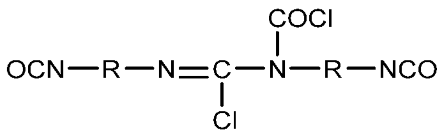

,

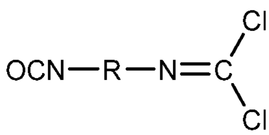

;

where R is a divalent group obtained by removing NCO group from isocyanate.

Preferably, R is selected from any one of

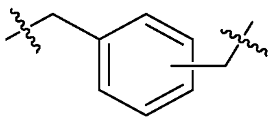

(isocyanate is XDI),

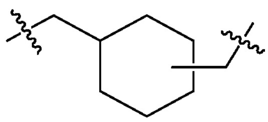

(isocyanate is H6XDI), and

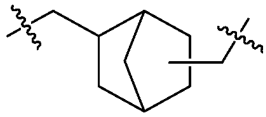

(isocyanate is NBDI), or a combination of at least two thereof, where a wavy line represents a linking site of groups.

Preferably, the chloroisocyanate is a compound obtained by replacing one NCO group of the isocyanate with chlorine.

Preferably, the chloroisocyanate includes any one of (chloromethyl benzyl isocyanate CBI, isocyanate is XDI),

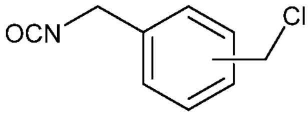

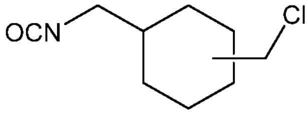

(chloromethyl methyl-isocyanato cyclohexane CIC, isocyanate is H6XDI), and

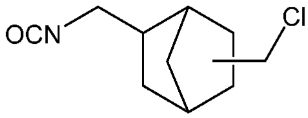

(chloromethyl methyl-isocyanato norborane CNBI, isocyanate is NBDI), or a combination of at least two thereof.

Herein, the expression of a ring structure crossed by "-" indicates that the linking site is at any position on the ring structure that is capable of forming a bond.

Preferably, the mass content of chloroisocyanate in isocyanate composition (B value) is 10 ppm to 2000 ppm, for example, it may be 20 ppm, 50 ppm, 100 ppm, 300 ppm, 500 ppm, 700 ppm, 900 ppm, 1000 ppm, 1100 ppm, 1300 ppm, 1500 ppm, 1700 ppm or 1900 ppm, or, specific point values therebetween. Considering limited space and brevity, the present application will not list exhaustively the specific point values encompassed by the ranges. More preferably, B value is 80 ppm to 1500 ppm.

It should be noted that, in the present application, the substance corresponding to the effective factor and chloroisocyanate may be produced as by-products in the preparation process of isocyanate, or may be artificially added to obtain the required content.

In a second aspect, embodiments of the present application provide a preparation method of the isocyanate composition according to the first aspect, where the preparation method includes reacting an amine compound with phosgene to obtain the isocyanate composition.

Preferably, the preparation method includes the following steps:

(1) reacting an amine compound with phosgene to obtain a reaction product;
(2) performing a removal treatment on the reaction product obtained in the step (1) to obtain a crude product, where the removal treatment includes phosgene removal treatment and/or solvent removal treatment;
(3) performing separation and refining on the crude product obtained in step (2) to obtain the isocyanate composition.

Preferably, a heavy component and an intermediate product are obtained by the separation in step (3); a mixture of the intermediate product and the heavy component is refined to obtain the isocyanate composition, where a mass percentage of the heavy component in the mixture is 1% to 10%.

As a preferred technical solution of the present application, the component to be refined is a mixture of the intermediate product and the heavy component, and the mass percentage of the heavy component in the mixture is 1% to 10%, for example, it may be 2%, 3%, 4%, 5%, 6%, 7%, 8% or 9%, and specific point values therebetween. Considering limited space and brevity, the present application will not list exhaustively the specific point values encompassed by the ranges. More preferably, the mass percentage of the heavy component in the mixture is 2% to 10%.

Preferably, the separated heavy component may be directly mixed with an intermediate product to obtain a mixture; or, the separated heavy component is primary heavy component, and the primary heavy component is separated again to obtain a heavy component recovery material and a residual heavy component; the heavy component recovery material is mixed with the intermediate product to obtain the mixture, and the heavy component recovery material in the

mixture has a mass percentage of 1% to 10%.

In another preferred technical solution, the preparation method of the isocyanate composition includes: mixing an isocyanate obtained by a urethane cleavage method with the heavy component recovery material to obtain the isocyanate composition. Preferably, the mass percentage of the heavy component recovery material in the isocyanate composition is 1% to 10% (e.g., 2%, 3%, 4%, 5%, 6%, 7%, 8%, or 9%, etc.), more preferably, 1% to 5%.

As a preferred technical solution of the present application, the preparation method of the isocyanate composition is phosgenation method, that is, the amine compound reacts with phosgene to form isocyanate; the amine compound includes diamine and/or diamine salt (for example, diamine hydrochloride obtained by the reaction of diamine with HCl).

Preferably, the method of reacting the amine compound with phosgene exemplarily includes the following three categories: a method of reacting diamine with phosgene in a gas phase, which is also called a gas phase phosgene method; a method of reacting diamine with phosgene in a liquid phase, which is also called a liquid phase phosgene method; and a method of reacting a diamine salt (e.g., diamine hydrochloride) with phosgene in a solvent, which is also called a phosgene method of diamine hydrochloride. More preferably, the method of reacting the amine compound with phosgene exemplarily includes a phosgene method of diamine hydrochloride.

Preferably, the amine compound in step (1) is diamine hydrochloride.

Preferably, the diamine hydrochloride is prepared by a salt formation process, and the salt formation process includes: subjecting diamine and hydrogen chloride to the salt formation reaction in the presence of a reaction solvent to obtain the diamine hydrochloride. A slurry containing diamine hydrochloride is actually obtained in the salt formation process, and the slurry is directly applied to the reaction with phosgene (isocyanation process).

Preferably, the salt formation process specifically includes: introducing hydrogen chloride gas into a reaction solvent, adding an amine solution (including a reaction solvent and diamine), stirring and mixing, and performing a salt formation reaction to obtain the diamine hydrochloride.

Preferably, the molar ratio of hydrogen chloride to diamine in the salt formation process is (2-20):1, for example, it may be 2.5:1, 3:1, 4:1, 5:1, 6:1, 7:1, 8:1, 9:1, 10:1, 11:1, 12:1, 13:1, 14:1, 15:1, 16:1, 17:1 or 18:1, etc., more preferably, (3-16):1.

Preferably, the mass percentage of diamine in the amine solution is 1 wt.% to 50 wt.%, for example, it may be 2 wt.%, 5 wt.%, 8 wt.%, 10 wt.%, 12 wt.%, 15 wt.%, 18 wt.%, 20 wt.%, 22 wt.%, 25 wt.%, 28 wt.%, 30 wt.%, 35 wt.%, 40 wt.%, 45 wt.% or 48 wt.%, and, specific point values therebetween. Considering limited space and brevity, the present application will not list exhaustively the specific point values encompassed by the ranges. More preferably, the mass percentage of diamine in the amine solution is 3 wt.% to 30 wt.%.

Preferably, the temperature of the salt formation reaction is 0 °C to 160 °C, for example, it may be 5 °C, 10 °C, 20 °C, 30 °C, 40 °C, 50 °C, 60 °C, 80 °C, 100 °C, 110 °C, 130 °C or 150 °C, and, specific point values therebetween. Considering limited space and brevity, the present application will not list exhaustively the specific point values encompassed by the ranges. More preferably, the temperature of the salt formation reaction is 10 °C to 150 °C. Even more preferably, the temperature of the salt formation reaction is 10 °C to 140 °C.

Preferably, the salt formation process is performed under a normal pressure or an elevated pressure.

Preferably, the pressure (gauge pressure) of the salt formation process is 0.01 MPa G to 1.0 MPa G, for example, it may be 0.03 MPa G, 0.05 MPa G, 0.08 MPa G, 0.1 MPa G, 0.2 MPa G, 0.3 MPa G, 0.4 MPa G, 0.5 MPa G, 0.6 MPa G, 0.7 MPa G, 0.8 MPa G or 0.9 MPa G, and, specific point values therebetween. Considering limited space and brevity, the present application will not list exhaustively the specific point values encompassed by the ranges. More preferably, the pressure (gauge pressure) of the salt formation process is 0.02 MPa G to 0.5 MPa G, even more preferably, 0.02 MPa G to 0.4 MPa G.

Preferably, step (1) specifically includes introducing phosgene into diamine hydrochloride (slurry) to carry out reaction to obtain a reaction product, i.e., a reaction solution containing diisocyanate.

Preferably, the molar ratio of phosgene to diamine hydrochloride is (4-50):1, for example, it may be 5:1, 8:1, 10:1, 12:1, 15:1, 18:1, 20:1, 22:1, 25:1, 28:1, 30:1, 32:1, 35:1, 38:1, 40:1, 42:1, 45:1 or 48:1, etc. More preferably, the molar ratio of phosgene to diamine hydrochloride is (5-40):1, even more preferably, (5-30):1.

Preferably, the temperature of the reaction in step (1) is 80 °C to 180 °C, for example, it may be 90 °C, 100 °C, 110 °C, 120 °C, 130 °C, 140 °C, 150 °C, 160 °C or 170 °C, and, specific point values therebetween. Considering limited space and brevity, the present application will not list exhaustively the specific point values encompassed by the ranges. More preferably, the temperature of the reaction in step (1) is 100 °C to 170 °C, even more preferably, 100 °C to 160 °C.

Preferably, the reaction time in step (1) is 2 h to 25 h, for example, it may be 3 h, 4 h, 6 h, 8 h, 10 h, 12 h, 14 h, 16 h, 18 h, 20 h, 22 h or 24 h, and, specific point values therebetween. Considering limited space and brevity, the present application will not list exhaustively the specific point values encompassed by the ranges. More preferably, the reaction time in step (1) is 4 h to 20 h.

Preferably, the reaction in the step (1) is performed under a normal pressure or an elevated pressure.

Preferably, the pressure (gauge pressure) of the reaction in the step (1) is 0 MPa G to 0.6 MPa G, for example, it may be 0.0005 MPa G, 0.001 MPa G, 0.003 MPa G, 0.01 MPa G, 0.02 MPa G, 0.03 MPa G, 0.05 MPa G, 0.07 MPa G, 0.09

MPa G, 0.1 MPa G, 0.2 MPa G, 0.3 MPa G, 0.4 MPa G or 0.5 MPa G, and, specific point values therebetween. Considering limited space and brevity, the present application will not list exhaustively the specific point values encompassed by the ranges. More preferably, the pressure (gauge pressure) of the reaction in the step (1) is 0.005 MPa G to 0.4 MPa G, even more preferably, 0.01 MPa G to 0.2 MPa G.

Preferably, the reaction (isocyanation process) in the step (1) is a batch process or a continuous process, preferably a continuous process.

The continuous process is that the slurry (diamine hydrochloride) produced in the salt formation process (salt formation kettle) is continuously transported from a stirring tank to a reaction kettle different from the salt formation kettle, the diamine hydrochloride is reacted with phosgene in the reaction kettle, and the obtained reaction product (a reaction solution containing diisocyanate) is continuously taken out from the reaction kettle. The number of the reaction kettle in the continuous process is not specifically limited in the present application, and exemplarily, may be 2, 3, 4, 5 or more.

As required, the reaction product obtained in the step (1) may be subjected to a removal process (solvent removal process and/or phosgene removal process) and a separation and refining process.

Preferably, the reaction solvent is an organic solvent, exemplarily, including but not limited to aromatic hydrocarbons such as benzene, toluene, and xylene; aliphatic hydrocarbons such as octane and decane; alicyclic hydrocarbons such as cyclohexane, methylcyclohexane, and ethylcyclohexane; halogenated aromatic hydrocarbons such as chlorotoluene, chlorobenzene, dichlorobenzene, dibromobenzene and trichlorobenzene; nitrogen-containing compounds such as nitrobenzene, N,N-dimethylformamide, N,N-dimethylacetamide, and N,N'-dimethylimidazolinone; ethers such as dibutyl ether, ethylene glycol dimethyl ether and ethylene glycol diethyl ether; ketones such as heptanone, diisobutyl ketone, methyl isobutyl ketone and methyl ethyl ketone; fatty acid esters such as ethyl acetate, butyl acetate, amyl acetate, and ethoxyethyl acetate; aromatic carboxylic ester such as methyl salicylate, dimethyl phthalate, dibutyl phthalate, and methyl benzoate. The reaction solvent may be used alone or in combination of at least two.

Preferably, the reaction solvent includes halogenated aromatic hydrocarbon, more preferably, chlorobenzene and/or dichlorobenzene.

Preferably, the phosgene removal treatment in the step (2) is performed in a phosgene removal tower.

Preferably, the solvent removal treatment of the step (2) is performed in a solvent removal tower.

Preferably, in the separation described in the step (3), the intermediate product (light component) and the heavy component are separated to achieve removal of the heavy component; a separation device exemplarily includes but is not limited to a short-path evaporator and a distillation column.

Preferably, the operating pressure of the short-path evaporator is 0.05 kPa to 4 kPa, for example, it may be 0.08 kPa, 0.1 kPa, 0.3 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa or 3.8 kPa, and, specific point values therebetween. Considering limited space and brevity, the present application will not list exhaustively the specific point values encompassed by the ranges. More preferably, the operating pressure of the short-path evaporator is 0.1 kPa to 2.5 kPa.

As a preferred technical solution of the present application, the heavy component obtained by separation contains kinds of chlorine-containing substances with high content. By blending the separated heavy component into the separated intermediate product (light component) in a certain proportion to obtain a mixture and then refining the mixture, the type and content of chlorine-containing substances in the product can be effectively controlled, so that the effective factor of the isocyanate composition is 3.90 to 5.70.

Preferably, the mass percentage of the heavy component (the heavy component recovery material) in the mixture is 1% to 10%, more preferably, 2% to 10%, so that the effective factor of the isocyanate composition is 3.90 to 5.70. If the blended amount of the heavy component (the heavy component recovery material) is too low, the effective factor will be too high, so that when the isocyanate composition is used to prepare polyurethane or polythiourethane, it is easy for occurrence of undesirable phenomena such as gelation. If the blended amount of the heavy component (the heavy component recovery material) is too high, the effective factor will be low, so that the isocyanate composition contains more impurities, which affects the discoloration (yellowing) resistance and stability of polyurethane/polythiourethane, resulting in poor optical properties of optical materials.

Preferably, the heavy component mixed with the intermediate product may be directly blended back into the intermediate product, or may be recycled and separated through a heavy component removal apparatus to obtain a heavy component recovery material, which is blended into the intermediate product again.

Preferably, the refining method is an industrial separation technique known in the art, exemplarily, it includes but is not limited to distillation, rectification, crystallization, etc.

Preferably, the refining method is rectification.

Preferably, the rectification is performed in a rectification tower, and the rectification tower preferably includes a plate rectification tower or a packed rectification tower.

Preferably, the theoretical plate number of the rectification tower is 2 to 60, for example, it may be 3, 5, 8, 10, 12, 15, 18, 20, 22, 25, 28, 30, 32, 35, 38, 40, 42, 45, 48, 52, 52, 55 or 58, and, specific point values therebetween. Considering limited space and brevity, the present application will not list exhaustively the specific point values encompassed by the

ranges. More preferably, the theoretical plate number of the rectification tower is 5 to 40.

Preferably, the top pressure of the rectification tower is 0.1 kPa to 4 kPa, for example, it may be 0.2 kPa, 0.5 kPa, 0.8 kPa, 1 kPa, 1.2 kPa, 1.5 kPa, 1.8 kPa, 2 kPa, 2.2 kPa, 2.5 kPa, 2.8 kPa, 3 kPa, 3.2 kPa, 3.5 kPa or 3.8 kPa, and, specific point values therebetween. Considering limited space and brevity, the present application will not list exhaustively the specific point values encompassed by the ranges. More preferably, the top pressure of the rectification tower is 0.15 kPa to 2.5 kPa.

Preferably, the top reflux ratio of the rectification tower is 0.01 to 60, for example, it may be 0.05, 0.1, 0.5, 1, 3, 5, 8, 10, 12, 15, 18, 20, 22, 25, 28, 30, 32, 35, 38, 40, 42, 45, 48, 50, 52, 55 or 58, and, specific point values therebetween. Considering limited space and brevity, the present application will not list exhaustively the specific point values encompassed by the ranges. More preferably, the top reflux ratio of the rectification tower is 0.1 to 40.

In one preferred technical solution of the present application, the preparation method of the isocyanate composition includes the following steps:

(1a) a salt formation process: subjecting diamine and hydrogen chloride to a salt formation reaction to obtain diamine hydrochloride;

(1) a phosgenation process: reacting the diamine hydrochloride obtained in the step (1) with phosgene to obtain a reaction product;

(2) a removal process: performing a removal treatment on the reaction product obtained in the step (1) to obtain a crude product, where the removal treatment includes a phosgene removal treatment and/or a solvent removal treatment;

(3a) a separation process: separating the crude product obtained in the step (2) to obtain a heavy component and an intermediate product (light component);

(3b) a heavy component recovery process: mixing the intermediate product and the heavy component obtained in the step (3a) to obtain a mixture, where a mass percentage of the heavy component in the mixture is 1% to 10%; or, performing secondary separation on the heavy component obtained in the step (3a) to obtain a heavy component recovery material and a residual heavy component, and mixing the heavy component recovery material with the intermediate product to obtain a mixture, where a mass percentage of the heavy component recovery material in the mixture is 1% to 10%;

(3c) a refining process: refining the mixture obtained in the step (3b) to obtain the isocyanate composition.

Exemplarily, the schematic flow diagram of the preparation method is shown in FIG. 1, and includes a salt formation process 10, a phosgenation process 20, a removal process 30, a separation process 40, a heavy component recovery process 50, and a refining process 60. Where, the salt formation process and the phosgenation process may be performed in a batch mode or a continuous mode, and the continuous mode is performed continuously in a kettle mode. The effective factor of the isocyanate composition is adjusted by appropriately adjusting the mixing ratio of the heavy component and the intermediate product, the feeding ratio of phosgene, the reaction temperature, the reaction pressure, the average residence time, the reflux ratio of the rectification tower, and the like; and the control of the effective factor is achieved mainly by the ratio of the heavy component and the intermediate product.

Specifically, taking the XDI composition as an example, the preparation method thereof is as follows.

(1a) A salt formation process: a kettle reaction is used, e.g., using a single kettle or two kettles. The process includes: first, charging a reaction solvent into a salt-forming kettle; then, adding hydrogen chloride and phenylenedimethylene diamine (XDA) into the salt-forming kettle at the above ratio of hydrogen chloride and diamine; stirring and mixing hydrogen chloride and amine solution with a stirring blade while maintaining the inside of the salt-forming tank at the above-mentioned salt-forming temperature and pressure to obtain XDA hydrochloride; continuously feeding hydrogen chloride gas and amine solution to the salt-forming kettle and at the same time continuously taking out a slurry containing the XDA hydrochloride from the salt-forming kettle and conveying the slurry to the phosgenation process.

(1) A phosgenation process: a kettle reaction is used, e.g., using three kettles or four kettles that are successive. The process includes: continuously feeding the above XDA hydrochloride into a phosgenation reaction kettle, continuously introducing phosgene into the respective tops of a first phosgenation kettle, a second phosgenation kettle and a third phosgenation kettle by means of an insertion pipe at the above feeding ratio; and then stirring and mixing the slurry and the phosgene while maintaining the inside of respective phosgenation kettles at the above-mentioned temperature and pressure; thus, reacting the XDA hydrochloride with carbonyl chloride to produce XDI as a main component, and produce CBI and other intermediates as by-products, obtaining a reaction solution.

Thus, the salt formation process and the phosgenation process are continuously performed.

(2) A removal process: a phosgene removal tower and a solvent removal tower are used. The process includes continuously conveying the above reaction solution to the middle part of the phosgene removal tower; removing phosgene, hydrogen chloride and the like from the reaction solution through the phosgene removal tower; and removing solvent from the reaction solution through the solvent removal tower to obtain a crude XDI product.

(3a) A separation process: separating the above crude XDI product by using a short-path evaporator and removing a heavy component to obtain an intermediate product and a primary heavy component.

(3b) A heavy component recovery process: recovering the primary heavy component by a short-path evaporator to obtain a heavy component recovery material and a secondary heavy component, which may be recovered in a single time or in a cycled manner; and mixing the heavy component recovery material with the intermediate product to obtain a mixture for a refining process, where the mass percentage of the heavy component recovery material in the mixture is 1% to 10%.

(3c) A refining process: continuously feeding the above mixture into a rectification tower, then distilling off low-boiling substances from the intermediate product under the above rectification conditions (bottom temperature, top temperature, top pressure, bottom reflux ratio, top reflux ratio, residence time), and recovering an XDI composition from the middle part of the tower.

Thus, the XDI composition including XDI, CBI, and substances corresponding to an effective factor may be continuously prepared.

In a third aspect, embodiments of the present application provide a modified isocyanate, which is obtained by modifying the isocyanate composition of the first aspect. The modified isocyanate includes any one of groups (a)-(i) or a combination of at least two thereof: (a) isocyanurate group, (b) uretidione group, (c) biuret group, (d) urethane group, (e) urea group, (f) iminooxadiazinedione group, (g) allophanate group, (h) uretonimine group, (i) carbodiimide group.

Those skilled in the art may modify the isocyanate composition by known methods to obtain the modified isocyanate according to the need. The modified isocyanate as a polyisocyanate component and an active hydrogen group-containing substance is suitably utilized as a raw material for polyurethane resin and polythiourethane resin.

Specifically, the modified isocyanate containing the group (a) isocyanurate group is a trimer of isocyanate, which may be obtained, for example, by reacting an isocyanate composition in the presence of a known isocyanurate-formation catalyst and causing the isocyanate therein to undergo isocyanurate-formation.

The modified isocyanate containing the group (b) uretidione group may be obtained by heating an isocyanate composition at about 90 °C to 200 °C or by reacting the isocyanate composition in the presence of a known uretidione-formation catalyst and causing the isocyanate to undergo uretidione-formation (e.g., dimerization).

The modified isocyanate containing the group (c) biuret group may be obtained by reacting an isocyanate composition with, for example, water, tertiary alcohol (e.g., tert-butyl alcohol), secondary amine (e.g., dimethylamine, diethylamine), and after the reaction, further reacting the isocyanate composition in the presence of a known biuret-formation catalyst.

The modified isocyanate containing the group (d) urethane group may be obtained by reacting an isocyanate composition with a polyol component (e.g., trimethylolpropane).

The modified isocyanate containing the group (e) urea group may be obtained by reacting an isocyanate composition with water, a polyamine component (described later), or the like.

The modified isocyanate containing the group (f) iminooxadiazinedione group, i.e., the asymmetric trimer of isocyanate, may be obtained by reacting an isocyanate composition in the presence of a known iminooxadiazinedione-formation catalyst and causing the isocyanate to undergo iminooxadiazinedione formation (e.g., trimerization).

The modified isocyanate containing the group (g) allophanate group may be obtained by reacting an isocyanate composition with an alcohol and further reacting it in the presence of a known allophanate-formation catalyst.

The modified isocyanate containing the group (h) uretonimine group may be obtained by reacting an isocyanate composition in the presence of a known carbodiimide-formation catalyst to form a carbodiimide group and then adding an isocyanate to the carbodiimide group.

The modified isocyanate containing the group (i) carbodiimide group may be obtained by reacting an isocyanate composition in the presence of a known carbodiimide-formation catalyst.

It should be noted that, the modified isocyanate may contain at least one or two of the groups (a) to (i) described above. Such modified isocyanate may be generated by appropriately combining the above-mentioned reactions. In addition, the modified isocyanate may be used alone or in combination of two or more of modified isocyanates.

In a fourth aspect, embodiments of the present application provide a polyurethane resin formed by reacting an isocyanate-based substance with an active hydrogen group-containing substance, where the isocyanate-based substance includes at least one of the isocyanate composition according to the first aspect and the modified isocyanate according to the third aspect.

Preferably, the active hydrogen group includes any one of hydroxyl group, amino group and mercapto group, or a combination of at least two thereof.

Preferably, the active hydrogen group-containing substance includes any one of polyol, polyamine and polythiol, or a combination of at least two thereof.

In a fifth aspect, the present application provides an optical material obtained by polymerizing an isocyanate-based substance with a polythiol compound, where the isocyanate-based substance includes at least one of the isocyanate composition according to the first aspect and the modified isocyanate according to the third aspect.

In the present application, the optical material is polythiourethane, and the polythiol compound is a compound containing at least two thiol groups (mercapto groups).

Preferably, the optical material is applied to eyeglasses, lens, prisms, lampshade materials, transparent packaging materials, transparent building materials or electronic products. Specifically, the optical material is applied to eyeglasses, plastic lens, prisms, automobile lampshade materials, transparent roof materials, lens material of electronic device (a mobile phone or a tablet computer) and the like.

Preferably, the polythiol compound includes an aliphatic polythiol compound such as methyldithiol, 1,2-ethanedithiol, 1,1-propanedithiol, 1,2-propanedithiol, 1,3-propanedithiol, 2,2-propanedithiol, 1,6-hexanedithiol, 1,2,3-propanetrithiol, 1,1-cyclohexanedithiol, 1,2-cyclohexanedithiol, 2,2-dimethylpropane-1,3-dithiol, 3,4-dimethoxybutane-1,2-dithiol, 2-methylcyclohexane-2,3-dithiol, 1,1-bis(mercaptomethyl)cyclohexane, bis(2-mercaptoethyl) thiomalate, 2,3-dimercapto-1-propanol(2-mercaptoacetate), 2,3-dimercapto-1-propanol(3-mercaptopropionate), diethylene glycol bis(2-mercaptoacetate), diethylene glycol bis(3-mercaptopropionate), 1,2-dimercaptopropyl methyl ether, 2,3-dimercaptopropyl methyl ether, 2,2-bis(mercaptomethyl)-1,3-propanedithiol, bis(2-mercaptoethyl) ether, ethylene glycol bis(2-mercaptoacetate), ethylene glycol bis(3-mercaptopropionate), trimethylolpropane bis(2-mercaptoacetate), trimethylolpropane bis(3-mercaptopropionate), pentaerythritol tetra(2-mercaptoacetate), pentaerythritol tetra (3-mercaptopropionate), and tetra(mercaptomethyl) methane.

Preferably, the polythiol compound further includes an aromatic polythiol compound such as 1,2-dimercaptobenzene, 1,3-dimercaptobenzene, 1,4-dimercaptobenzene, 1,2-bis(mercaptomethyl) benzene, 1,3-bis(mercaptomethyl) benzene, 1,4-bis(mercaptomethyl) benzene, 1,2-bis(mercaptoethyl) benzene, 1,3-bis(mercaptoethyl) benzene, 1,4-bis(mercaptoethyl) benzene, 1,2,3-trimercaptobenzene, 1,2,4-trimercaptobenzene, 1,3,5-trimercaptobenzene, 1,2,3-tris(mercaptomethyl) benzene, 1,2,4-tris(mercaptomethyl) benzene, 1,3,5-tris(mercaptomethyl) benzene, 1,2,3-tris(mercaptoethyl) benzene, 1,2,4-tris(mercaptoethyl) benzene, 1,3,5-tris(mercaptoethyl) benzene, 2,5-toluenedithiol, 3,4-toluenedithiol, 1,3-bis(p-methoxyphenyl) propane-2,2-dithiol, 1,3-diphenylpropane-2,2-dithiol, phenylmethane-1,1-dithiol, and 2,4-bis(p-mercaptophenyl) pentane.

Preferably, the polythiol compound further includes an aromatic polythiol compound containing sulfur atom besides mercapto group, such as 1,2-bis(mercaptoethylthio) benzene, 1,3-bis(mercaptoethylthio) benzene, 1,4-bis(mercaptoethylthio) benzene, 1,2,3-tris(mercaptomethylthio) benzene, 1,2,4-tris(mercaptomethylthio) benzene, 1,3,5-tris(mercaptomethylthio) benzene, 1,2,3-tris(mercaptoethylthio) benzene, 1,2,4-tris(mercaptoethylthio) benzene, 1,3,5-tris(mercaptoethylthio) benzene, and alkylate thereof.

Preferably, the polythiol compound further includes an aliphatic polythiol compound containing sulfur atom besides mercapto group, such as bis(mercaptomethyl) sulfide, bis(mercaptomethyl) disulfide, bis(mercaptoethyl) sulfide, bis(mercaptoethyl) disulfide, bis(mercaptopropyl) sulfide, bis(mercaptomethylthio) methane, bis(2-mercaptoethylthio) methane, bis(3-mercaptopropylthio) methane, 1,2-bis(mercaptomethylthio) ethane, 1,2-bis(2-mercaptoethylthio) ethane, 1,2-bis(3-mercaptopropyl) ethane, 1,3-bis(mercaptomethylthio) propane, 1,3-bis(2-mercaptoethylthio) propane, 1,3-bis(3-mercaptopropylthio) propane, 1,2,3-tri(mercaptomethylthio) propane, 1,2,3-tri(2-mercaptoethylthio) propane, 1,2,3-tri(3-mercaptopropylthio) propane, 1,2-bis((2-mercaptoethyl)thio)-3-mercaptopropane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, bis(mercaptomethyl)-3,6,9-trithia-1,11-undecane dithiol, tetrakis(mercaptomethylthiomethyl) methane, tetrakis(2-mercaptoethylthiomethyl) methane, tetrakis(3-mercaptopropylthiomethyl) methane, bis(2,3-dimercaptopropyl) sulfide, bis(1,3-dimercaptopropyl) sulfide, 2,5-dimercapto-1,4-dithiane, 2,5-dimercapto methyl-1,4-dithiane, 2,5-dimercapto methyl-2,5-dimethyl-1,4-dithiane, bis(mercaptomethyl) disulfide, bis(mercaptoethyl) disulfide and bis(mercaptopropyl) disulfide, and esters of thioglycolic acid and mercaptopropionic acid thereof.

Preferably, the polythiol compound further includes other aliphatic polythiol compound containing sulfur atom and ester bond besides mercapto group, such as hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxypropyl sulfide bis(2-mercaptoacetate), hydroxypropyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), hydroxypropyl disulfide bis(2-mercaptoacetate), hydroxypropyl disulfide bis(3-mercaptopropionate), 2-mercaptoethyl ether bis(2-mercaptoacetate), 2-mercaptoethyl ether bis(3-mercaptopropionate), 1,4-dithiane-2,5-diol bis(2-mercaptoacetate), 1,4-dithiane-2,5-diol bis(3-mercaptopropionate), thiodiacetate bis(2-mercaptoacetate), thiodipropionate bis(2-mercaptoacetate), 4,4-thiodibutyrate bis(2-mercaptoacetate), dithiodiacetate bis(2-mercaptoacetate), dithiodipropionate bis(2-mercaptoacetate), 4,4-dithiodibutyrate bis(2-mercaptoacetate), thiodiacetate bis(2,3-dimercaptopropyl ester), thiodipropionate bis(2,3-dimercaptopropyl ester), dithiodiacetate bis(2,3-dimercaptopropyl ester), dithiodipropionate bis(2,3-dimercaptopropyl ester).

Preferably, the polythiol compound further includes a heterocyclic compound containing sulfur atom besides mercapto group, such as 3,4-thienedithiol and 2,5-dimercapto-1,3,4-thiadiazole.

Preferably, the polythiol compound further includes a compound containing hydroxyl group besides mercapto group, such as 2-mercaptoethanol, 3-mercapto-1,2-propanediol, glycerol bis(mercaptoacetate), 1-hydroxy-4-mercaptocyclohexane, 2,4-dimercaptophenol, 2-mercaptohydroquinone, 4-mercaptophenol, 3,4-dimercapto-2-propanol, 1,3-dimercapto-2-propanol, 2,3-dimercapto-1-propanol, 1,2-dimercapto-1,3-butanediol, pentaerythritol tris(3-mercaptopropionate), pentaerythritol mono(3-mercaptopropionate), pentaerythritol bis(3-mercaptopropionate), pentaerythritol tri(mercaptoacetate), dipentaerythritol penta(3-mercaptopropionate), hydroxymethyl-tris(mercaptoethylthiomethyl) methane, 1-hydroxyethylthio-3-mercaptoethylthio benzene and the like.

Preferably, the polythiol compound further includes a compound having dithioacetal or dithioketal skeleton, such as 1,1,3,3-tetrakis(mercaptomethylthio) propane, 1,1,2,2-tetrakis(mercaptomethylthio) ethane, 4,6-bis(mercaptomethylthio)-1,3-dithiacyclohexane, 1,1,5,5-tetrakis(mercaptomethylthio)-3-thiapentane, 1,1,6,6-tetrakis(mercaptomethylthio)-3,4-dithiahexane, 2,2-bis(mercaptomethylthio) ethanethiol, 2-(4,5-dimercapto-2-thiapentyl)-1,3-dithiacyclopentane, 2,2-bis(mercaptomethyl)-1,3-dithiacyclopentane, 2,5-bis(4,4-bis(mercaptomethylthio)-2-thiabutyl)-1,4-dithiane, 2,2-bis(mercaptomethylthio)-1,3-propanedithiol, 3-mercaptomethylthio-1,7-dimercapto-2,6-dithiaheptane, 3,6-bis(mercaptomethylthio)-1,9-dimercapto-2,5,8-trithianonane, 4,6-bis(mercaptomethylthio)-1,9-dimercapto-2,5,8-trithianonane, 3-mercaptomethylthio-1,6-dimercapto-2,5-dithiahexane, 2-(2,2-bis(mercaptomethylthio)ethyl)-1,3-dithiacyclobutane, 1,1,9,9-tetrakis(mercaptomethylthio)-5-(3,3-bis(mercaptomethylthio)-1-thiapropyl) 3,7-dithianonane, tri(2,2-bis(mercaptomethylthio)ethyl)methane, tri(4,4-bis(mercaptomethylthio)-2-thiabutyl) methane, tetrakis(2,2-bis(mercaptomethylthio) ethyl) methane, tetrakis(4,4-bis(mercaptomethylthio)-2-thiabutyl) methane, 3,5,9,11-tetrakis(mercaptomethylthio)-1,13-dimercapto-2,6,8,12-tetrathiatridecane, 3,5,9,11,15,17-hexa(mercaptomethylthio)-1,19-dimercapto-2,6,8,12,14,18-hexathia-nonadecane, 9-(2,2-bis(mercaptomethylthio)ethyl)-3,5,13,15-tetrakis(mercaptomethylthio)-1,17-dimercapto-2,6,8,10,12,16-hexathiaheptadecane, 3,4,8,9-tetrakis(mercaptomethylthio)-1,11-dimercapto-2,5,7,10-tetrathiaundecane, 3,4,8,9,13,14-hexa(mercaptomethylthio)-1,16-dimercapto-2,5,7,10,12,15-hexathiahexadecane, 8-(bis(mercaptomethylthio)methyl)-3,4,12,13-tetra(mercaptomethylthio)-1,15-dimercapto-2,5,7,9,11,14-hexathiapentadecane, 4,6-bis(3,5-bis(mercaptomethylthio)-7-mercapto-2,6-dithiaheptylthio)-1,3-dithiane, 4-(3,5-bis(mercaptomethylthio)-7-mercapto-2,6-dithiaheptylthio)-6-mercaptomethylthio-1,3-dithiane, 1,1-bis(4-(6-mercaptomethylthio)-1,3-dithianylthio)-3,3-bis(mercaptomethylthio) propane, 1,3-bis(4-(6-mercaptomethylthio)-1,3-dithianylthio)-1,3-bis(mercaptomethylthio) propane, 1-(4-(6-mercaptomethylthio)-1,3-dithianylthio)-3-(2,2-bis(mercaptomethylthio) ethyl)-7,9-bis(mercaptomethylthio)-2,4,6,10-tetrathiaundecane, 1-(4-(6-mercaptomethylthio)-1,3-dithianylthio)-3-(2-(1,3-dithiacyclobutyl))methyl-7,9-bis(mercaptomethylthio)-2,4,6,10-tetrathiaundecane, 1,5-bis(4-(6-mercaptomethylthio)-1,3-dithianylthio)-3-(2-(1,3-dithiacyclobutyl))methyl-2,4-dithiapentane, 4,6-bis(3-(2-(1,3-dithiacyclobutyl)) methyl-5-mercapto-2,4-dithiapentylthio)-1,3-dithiane, 4,6-bis(4-(6-mercaptomethylthio)-1,3-dithianylthio)-1,3-dithiane, 4-(4-(6-mercaptomethylthio)-1,3-dithianylthio)-6-(4-(6-mercaptomethylthio)-1,3-dithianylthio)-1,3-dithiane, 3-(2-(1,3-dithiacyclobutyl))methyl-7,9-bis(mercaptomethylthio)-1,11-dimercapto-2,4,6,10-tetrathiaundecane, 9-(2-(1,3-dithiacyclobutyl)) methyl-3,5,13,15-tetrakis (mercaptomethylthio)-1,17-dimercapto-2,6,8,10,12,16-hexathiaheptadecane, 3-(2-(1,3-dithiacyclobutyl))methyl-7,9,13,15-tetrakis(mercaptomethylthio)-1,17-dimercapto-2,4,6,10,12,16-hexathiaheptadecane, 3,7-bis(2-(1,3-dithiacyclobutyl)) methyl-1,9-dimercapto-2,4,6,8-tetrathianonane, 4-(3,4,8,9-tetrakis(mercaptomethylthio)-11-mercapto-2,5,7,10-tetrathiaundecyl)-5-mercaptomethylthio-1,3-dithiacyclopentane, 4,5-bis(3,4-bis(mercaptomethylthio)-6-mercapto-2,5-dithiahexylthio)-1,3-dithiacyclopentane, 4-(3,4-bis(mercaptomethylthio)-6-mercapto-2,5-dithiahexylthio)-5-mercaptomethylthio-1,3-dithiacyclopentane, 4-(3-bis(mercaptomethylthio)methyl-5,6-bis(mercaptomethylthio)-8-mercapto-2,4,7-trithiaoctyl)-5-mercaptomethylthio-1,3-dithiacyclopentane, 2-(bis(3,4-bis(mercaptomethylthio)-6-mercapto-2,5-dithiahexylthio)methyl)-1,3-dithiacyclobutane, 2-(3,4-bis(mercaptomethylthio)-6-mercapto-2,5-dithiahexylthio)mercaptomethylthio methyl-1,3-dithiacyclobutane, 2-(3,4,8,9-tetra(mercaptomethylthio)-11-mercapto-2,5,7,10-tetrathiaundecanethio)mercaptomethylthio methyl-1,3-dithiacyclobutane, 2-(3-bis(mercaptomethylthio)methyl-5,6-bis(mercaptomethylthio)-8-mercapto-2,4,7-trithiaoctyl)mercaptomethylthio methyl-1,3-dithiacyclobutane, 4,5-bis(1-(2-(1,3-dithiacyclobutyl))-3-mercapto-2-thiapropylthio)-1,3-dithiacyclopentane, 4-(1-(2-(1,3-dithiacyclobutyl))-3-mercapto-2-thiapropylthio)-5-(1,2-bis( mercaptomethylthio)-4-mercapto-3-thiabutylthio)-1,3-dithiacyclopentane, 2-(bis(4-(5-mercaptomethylthio-1,3-dithiolanyl)thio))methyl-1,3-dithiacyclobutane, 4-(4-(5-mercaptomethylthio-1,3-dithiolanyl)thio)-5-(1-(2-(1,3-dithiacyclobutyl)-3-mercapto-2-thiapropylthio)-1,3-dithiacyclopentane and oligomers thereof.

Preferably, the polythiol compound further includes a compound having a trithioorthoformate skeleton, such as tri(mercaptomethylthio) methane, tri(mercaptoethylthio) methane, 1,1,5,5-tetrakis(mercaptomethylthio)-2,4-dithiapentane, bis(4,4-bis(mercaptomethylthio)-1,3-dithiabutyl)(mercaptomethylthio) methane, tri(4,4-bis(mercaptomethylthio)-1,3-dithiabutyl) methane, 2,4,6-tri(mercaptomethylthio)-1,3,5-trithiacyclohexane, 2,4-bis(mercaptomethylthio)-1,3,5-trithiacyclohexane, 1,1,3,3-tetrakis(mercaptomethylthio)-2-thiapropane, bis(mercaptomethyl) methylthio-1,3,5-trithiacyclohexane, tri((4-mercaptomethyl-2,5-dithiacyclohexyl-1-yl) methylthio) methane, 2,4-bis(mercaptomethylthio)-1,3-dithiacyclopentane, 2-mercaptoethylthio-4-mercaptomethyl-1,3-dithiacyclopentane, 2-(2,3-dimercaptopropylthio)-1,3-dithiacyclopentane, 4-mercaptomethyl-2-(2,3-dimercaptopropylthio)-1,3-dithiacyclopentane, 4-mercaptomethyl-2-(1,3-dimercapto-2-propylthio)-1,3-dithiacyclopentane, tri(2,2-bis(mercaptomethylthio)-1-thiaethyl) methane, tri(3,3-bis(mercaptomethylthio)-2-thiapropyl) methane, tri(4,4-bis(mercaptomethylthio)-3-thiabutyl) methane, 2,4,6-tri(3,3-bis(mercaptomethylthio)-2-thiapropyl)-1,3,5-trithiacyclohexane, tetra(3,3-bis(mercaptomethylthio)-2-thiapropyl) methane, and oligomers thereof.

Preferably, the polythiol compound further includes a compound having a tetrathioorthocarbonate skeleton, such as 3,3'-bis(mercaptomethylthio)-1,5-dimercapto-2,4-dithiapentane, 2,2'-bis(mercaptomethylthio)-1,3-dithiacyclopentane, 2,7-bis(mercaptomethyl)-1,4,5,9-tetrathiaspiro[4,4] nonane, 3,9-dimercapto-1,5,7,11-tetrathiaspiro[5,5] undecane, and oligomers thereof.

It should be noted that the polythiol compound is not limited to the compounds mentioned above. Each of the above-mentioned compounds may be used alone or in combination of at least two.

Preferably, the polythiol compound includes at least one of 1,2-bis((2-mercaptoethyl)thio)-3-mercaptopropane, bis(mercaptomethyl)-3,6,9-trithia-1,11-undecanedithiol, pentaerythritol tetra(3-mercaptopropionate), 1,1,3,3-tetrakis (mercaptomethylthio)propane, or 2-mercaptoethanol.

Preferably, in the preparation of the optical material, the polymerization is performed in the presence of a polymerization catalyst, and the polymerization catalyst is preferably an organotin-based compound, which exemplarily includes, but is not limited to, dialkyltin halides such as dibutyltin dichloride and dimethyltin dichloride; dialkyltin dicarboxylic acids such as dimethyltin diacetate, dibutyltin dioctoate and dibutyltin dilaurate.

In addition, according to purposes, the preparation raw materials of the optical material further include optional auxiliary agent, which exemplarily includes but is not limited to any one of a chain extender, a crosslinking agent, a light stabilizer, a ultraviolet absorber, an antioxidant, an oil-soluble dye, a filler and a mold release agent, or a combination of at least two thereof.

Preferably, the optical material formed from a polythiourethane resin is generally manufactured using injection polymerization. In particular, the polythiol compound and the isocyanate-based substance are mixed, and optionally suitable auxiliary is added. If necessary, the mixture (polymerizable composition) is defoamed by an appropriate method, then injected into an injection mold for optical material, and usually heated slowly from a low temperature to a high temperature for polymerization. Then, the optical material is obtained by demoulding.

Preferably, the polymerization temperature is 80 °C to 200 °C, for example, it may be 90 °C, 100 °C, 110 °C, 120 °C, 130 °C, 140 °C, 150 °C, 160 °C, 170 °C, 180 °C, 190 °C, etc.

Preferably, the polymerization time is 1 h to 24 h, for example, it may be 2 h, 4 h, 6 h, 8 h, 10 h, 11 h, 12 h, 14 h, 16 h, 18 h, 20 h, 22 h or 23 h, etc.

As a preferred technical solution of the present application, the isocyanate composition is used for preparing optical material polythiourethane, which enables the optical material to have excellent transparency, color stability and yellowing resistance, and significantly reduces the optical deformation occurrence rate (≤1%, 0-1%) of the optical material. Specifically, the yellowness index YI of the optical material prepared with XDI composition is less than or equal to 1.60, and the YI of the optical material prepared with H6XDI composition or NBDI composition is less than or equal to 1.50.

As a preferred technical solution of the present application, the transmittance of the optical material (polythiourethane) prepared with the XDI composition is 83.95% to 84.10%, the transmittance of the optical material (polythiourethane) prepared with the H6XDI composition is greater than or equal to 86.0%, and the transmittance of the optical material (polythiourethane) prepared with the NBDI composition is 85.89% to 86.85%.

As a preferred technical solution of the present application, the glass transition temperature (Tg) of the optical material (polythiourethane) prepared with the XDI composition is 84.56 °C to 85.67 °C, the glass transition temperature of the optical material (polythiourethane) prepared with the H6XDI composition is 89.08 °C to 89.67 °C, and the glass transition temperature of the optical material (polythiourethane) prepared with the NBDI composition is 108.08 °C to 108.65 °C.

Compared with the related art, the embodiments of the present application have the following beneficial effects.

In the isocyanate composition provided by embodiments of the present application, by the design and control of effective factor, the isocyanate composition has excellent reactivity, and can be used for the preparation of high-performance resins such as polyurethane and polythiourethane without causing undesirable phenomena such as gelation during polymerization. In addition, the isocyanate composition can effectively improve the yellowing resistance, stability and optical performance of polyurethane, polythiourethane and other products, so that the obtained polyurethane and polythiourethane will not exhibit white turbidity and opacity phenomena. The optical material prepared from the isocyanate composition has high glass transition temperature, good heat resistance, remarkably reduced yellowness index and remarkably reduced optical deformation occurrence rate, and high transmittance, thereby effectively improving the optical

properties of the optical material.

Other aspects will become apparent upon reading and understanding the accompanying drawings and detailed description.

## BRIEF DESCRIPTION OF DRAWINGS

The accompanying drawings are used to provide a further understanding of the technical solutions herein and form a part of the description, and are intended to explain the technical solutions herein together with the embodiments of the present application, and do not constitute limitations on the technical solutions herein.

FIG. 1 is a schematic flow diagram of a method for preparing an isocyanate composition according to a specific implementation of the present application.
10 - salt formation process; 20 - phosgenation process; 30 - removal process; 40 - separation process; 50 - heavy component recovery process; 60 - refining process.

## DESCRIPTION OF EMBODIMENTS

The technical solution of the present application is further described below through specific implementations. It should be understood by those skilled in the art that the embodiments are only for facilitating understanding of the present application and should not be taken as specific limitations to the present application.

In the present application, the testing methods for components and properties are as follows.

1. Determination of a mass content of chlorine in an isocyanate composition (A value): XRF test.

Instrument: Energy dispersive X-ray fluorescence spectroscopy (ED-XRF), model: MERAK-LE II.

Method: Standard addition method.

Principle and operation: chromatographically pure $CCl_4$ standard is used as Cl source, ethyl acetate is used as a diluent, Cl element in a sample is excited by X-ray generated by an X-ray tube and characteristic X-ray fluorescence is generated, characteristic X-ray fluorescence intensity is linear with element concentration and a standard curve is drawn, where an extrapolated value is the content of Cl element in the sample.

2. Determination of a mass content of chloroisocyanate in an isocyanate composition (B value): GCMS test

An analysis is performed by gas chromatography-mass spectrometry under the following conditions, and the content herein is normalized content.

Analytical instrument: Agilent 5977B GCMS
Chromatographic column: DB-5 chromatographic column; specification: 30 m×0.25 mm×0.25 μm
Column temperature: holding at 50 °C for 2 min, then increasing the temperature to 80 °C at a rate of 5 mL/min, followed by increasing the temperature to 280 °C at a rate of 15 mL/min, and holding at 280 °C for 10 min.
Split ratio: splitless
Injection port temperature: 280 °C
Detection temperature: 300 °C
Carrier gas: helium
Carrier gas flow rate: 1 mL/min (constant flow)
Injection volume: 1 μL
Detection method: SIM selective ion monitoring mode (XDI selective ions are 160/126, H6XDI selective ions are 186/152, NBDI selective ions are 198/164)

3. Determination of a mass percentage of isocyanate in an isocyanate composition: gas chromatography test

An analysis is performed by gas chromatography under the following conditions, and the content herein is normalized content.

Analytical instrument: Agilent 7890B GC
Chromatographic column: DB-5 chromatographic column; specification: 30 m×0.25 mm×0.25 μm
Column temperature: holding at 60 °C for 1 min, then increasing the temperature at a rate of 10 °C/min to 300 °C, and holding at 300 °C for 5 min.
Split ratio: 30:1

Injection port temperature: 280 °C
Detection temperature: 320 °C
Carrier gas: nitrogen
Carrier gas flow: 1 mL/min (constant flow)
Injection volume: 1 μL
Detector: FID

In the specific embodiments of the present application, unless otherwise specified, "part" and "%" are based on mass.

Example 1

Provided are a composition of XDI and preparation method thereof, where the XDI composition has an effective factor E of 4.70 and a schematic flow diagram of its preparation method is shown in FIG. 1. The preparation method specifically includes the following steps.

A salt formation process: 800 parts by mass of chlorobenzene were put into a salt-forming kettle, where a salting temperature inside the salt-forming kettle was adjusted to 25 °C and a salting pressure (gauge pressure) inside the salt-forming kettle was adjusted to 0.04 MPa G. HCl gas was continuously blown into the salt-forming kettle at a rate of 85.8 parts by mass per hour, and an amine solution of 1,3-XDA (1,3-phenylenedimethylene diamine) (a mass concentration of 1,3-XDA was 8.0 wt.%, and the solvent was chlorobenzene) was continuously put into the salt-forming kettle at a rate of 1000 parts by mass per hour, so that the molar ratio of HCl to 1,3-XDA was 4:1. As a result, a slurry containing 1,3-XDA hydrochloride was generated and conveyed to a phosgenation kettle.

A phosgenation process: the slurry containing 1,3-XDA hydrochloride was transported to the phosgenation kettle, and the phosgene was continuously introduced into the phosgenation kettle at a rate of 581.5 parts by mass per hour. In the kettle, a reaction temperature was 145 °C and a reaction pressure (gauge pressure) was 0.2 MPa G, a molar ratio of the phosgene to 1,3-XDA hydrochloride was 10:1, and a residence time was 7 h. As a result, 1,3-XDA hydrochloride reacted with phosgene to produce 1,3-XDI, obtaining a reaction product containing 1,3-XDI.

A removal process: the reaction product obtained from the phosgenation process was continuously conveyed to a phosgene removal tower and a solvent removal tower for phosgene removal treatment and solvent removal treatment respectively. As a result, 108 parts by mass of 1,3-XDI crude product were obtained.

A separation process: the crude product was continuously conveyed to a short-path evaporator (a heavy component remover) to obtain 99.7 parts by mass of an intermediate product with heavy component removed and 6.3 parts by mass of a primary heavy component.

A heavy component recovery process: the primary heavy component was continuously conveyed to a secondary short-path evaporator to obtain 2.03 parts by mass of heavy component recovery material and 4.27 parts by mass of residual heavy component. The intermediate product with a rate of 99.7 parts by mass per hour and the heavy component recovery material with a rate of 2.03 parts by mass per hour were mixed to obtain a mixture, that is, the mass percentage of the heavy component recovery material in the mixture was 2 %.

A refining process: the aforementioned mixture was continuously fed into a rectification tower. The rectification tower was filled with a filler equivalent to a theoretical plate number of 25. Then, in the rectification tower, light components were removed from the top of the tower, and the XDI composition was collected from the middle of the tower to obtain a target product.

The rectification conditions in the rectification tower were as follows.

Tower bottom temperature: 145 °C to 150 °C
Tower top temperature: 100 °C to 120 °C
Tower top pressure: 0 PaA to 50 PaA
Residence time: 2 h to 4 h
Tower top reflux ratio: 10
Yield of the rectification process: 96.2 parts by mass per hour.

Thus, the XDI composition was obtained, where a mass content of 1,3-XDI was greater than 99%, a mass content of chlorine (A value) was 108 ppm, and a mass content of CBI (B value) was 450 ppm, and an effective factor E was 4.70.

Examples 2-5 and Comparative examples 1-2

Provided are an XDI composition and a preparation method thereof, where effective factors E of the XDI composition are shown in Table 1, respectively. The flow of the preparation method is the same as that in Example 1, except

for some different process parameters, which are specifically shown in Table 1 (the process/parameters that are not shown in Table 1 are exactly the same as those of Example 1). In Table 1, the "HCl ratio" indicates a molar amount of the HCl in the salt formation process based on 1 mol of 1,3-XDA; the "phosgene ratio" indicates a molar amount of the phosgene in the phosgenation process based on 1 mol of 1,3-XDA hydrochloride; and the "heavy component recovery material ratio" indicates a mass percentage of the heavy component (recovery material) in the mixture in the heavy component recovery process.

Table 1

| | | Example 1 | Example 2 | Example 3 | Example 4 | Example 5 | Comparative example 1 | Comparative example 2 |
|---|---|---|---|---|---|---|---|---|
| Salt formation process | HCl feeding rate (parts by mass per hour) | 85.8 | 107.2 | 128.6 | 150.1 | 214.4 | 214.4 | 85.8 |
| | HCl ratio | 4 | 5 | 6 | 7 | 10 | 10 | 4 |
| Phosgenation process | Phosgene feeding rate (parts by mass per hour) | 581.5 | 581 | 726.9 | 726.9 | 1133.9 | 1133.9 | 581.5 |
| | Phosgene ratio | 10 | 10 | 12.5 | 12.5 | 19.5 | 19.5 | 10 |
| | Reaction temperature( °C) | 145 | 146 | 147 | 148 | 149 | 149 | 145 |
| | Reaction pressure (MPa G) | 0.2 | 0.2 | 0.2 | 0.2 | 0.2 | 0.2 | 0.2 |
| | Residence time (h) | 7 | 7 | 7 | 7 | 10 | 10 | 7 |
| Heavy component recovery process | Mixture (parts by mass per hour) | 101.7 | 101.5 | 101 | 100 | 99 | 99 | 101.7 |
| | Heavy component recovery material (parts by mass per hour) | 2.03 | 3.56 | 5.76 | 7.6 | 9.9 | 11.76 | 0.51 |
| | Heavy component recovery material ratio (%) | 2 | 3.5 | 5.7 | 7.6 | 10 | 12 | 0.5 |
| Refining process | Withdrawal rate (parts by mass per hour) | 96.2 | 96.3 | 95.7 | 94.6 | 94.0 | 94.0 | 96.2 |
| | Reflux ratio | 10 | 8 | 8 | 6 | 5.5 | 5.5 | 10 |
| XDI composition | 1,3-XDI (%) | >99 | >99 | >99 | >99 | >99 | >99 | >99 |
| | Chlorine (ppm) | 108 | 150 | 230 | 330 | 421.6 | 440 | 89 |
| | CBI (ppm) | 450 | 505 | 723 | 1153 | 1517 | 1517 | 450 |
| | Effective factor E | 4.70 | 4.29 | 4.05 | 3.98 | 3.90 | 3.84 | 6.00 |

Examples 6-10 and Comparative examples 3-4

Provided are an H6XDI composition and a preparation method thereof, where effective factors E of the H6XDI composition are shown in Table 2 respectively. The process of the preparation method is the same as that in Example 1, except that the 1,3-XDA in the salt formation process is replaced by 1,3-H6 XDA, and the feeding amounts are kept the same. The feeding amounts are each 1000 parts by mass of amine solution of 1,3-H6 XDA (1,3-cyclohexyl dimethylamine) (the mass concentration is 8.0 wt.%, and the solvent is chlorobenzene) per hour. Some of the process parameters are

different and are specifically shown in Table 2 (the process and parameters that are not shown in Table 2 are exactly the same as those of Example 1). In Table 2, the "HCl ratio" indicates a molar amount of the HCl in the salt formation process based on 1 mol of 1,3-H6XDA; the "phosgene ratio" indicates a molar amount of the phosgene in the phosgenation process based on 1 mol of 1,3-H6XDA hydrochloride; and the "heavy component recovery material ratio" indicates a mass percentage of the heavy component (recovery material) in the mixture in the heavy component recovery process.

Table 2

| | | Example 6 | Example 7 | Example 8 | Example 9 | Example 10 | Comparative example 3 | Comparative example 4 |
|---|---|---|---|---|---|---|---|---|
| Salt formation process | HCl feeding rate (parts by mass per hour) | 82.2 | 102.7 | 123.2 | 143.8 | 205.4 | 205.4 | 82.2 |
| | HCl ratio | 4 | 5 | 6 | 7 | 10 | 10 | 4 |
| Phosgenation process | Phosgene feeding rate (parts by mass per hour) | 557.0 | 556.5 | 696.3 | 696.3 | 1086.1 | 1086.1 | 557.0 |
| | Phosgene ratio | 10 | 10 | 12.5 | 12.5 | 19.5 | 19.5 | 10 |
| | Reaction temperature( °C) | 148 | 149 | 150 | 151 | 152 | 152 | 148 |
| | Reaction pressure (MPa G) | 0.2 | 0.2 | 0.2 | 0.2 | 0.2 | 0.2 | 0.2 |
| | Residence time(h) | 9 | 9 | 9 | 9 | 12 | 12 | 9 |
| Heavy component recovery process | Mixture(parts by mass per hour) | 100.6 | 100.4 | 99.9 | 98.9 | 97.9 | 97.9 | 100.6 |
| | Heavy component recovery material(parts by mass per hour) | 2.0 | 3.5 | 5.7 | 7.5 | 9.8 | 11.8 | 0.5 |
| | Heavy component recovery material ratio (%) | 2 | 3.5 | 5.7 | 7.6 | 10 | 12 | 0.5 |
| Refining process | Withdrawal rate (parts by mass per hour) | 95.2 | 95.3 | 94.7 | 93.6 | 93.0 | 93.0 | 95.2 |
| | Reflux ratio | 10 | 8 | 8 | 6 | 5.5 | 5.5 | 10 |
| H6XDI composition | 1,3-H6XDI (%) | >99 | >99 | >99 | >99 | >99 | >99 | >99 |
| | Chlorine (ppm) | 25.6 | 64.7 | 143.9 | 234.3 | 352.5 | 369 | 24.6 |
| | CIC (ppm) | 125 | 210 | 465 | 853 | 1200 | 1200 | 125 |
| | Effective factor E | 5.70 | 4.60 | 4.25 | 4.14 | 3.90 | 3.85 | 6.00 |

Examples 11-15 and Comparative examples 5-6

Provided are an NBDI composition and a preparation method thereof. The effective factors E of the NBDI composition are shown in Table 3, respectively. The preparation method is the same as that of Example 1, except that the 1,3-XDA in the salt formation step is replaced by NBDA, and the feeding amounts are kept the same, all of which are 1000 parts by mass of amine solution of 1,3-NBDA (norbornane dimethylamine)(the mass concentration is 8.0 wt.%, and the solvent is chlorobenzene) per hour. Some of the process parameters are different and are specifically shown in Table 3 (the process/parameters not shown in Table 3 are exactly the same as those of Example 1). In Table 3, the "HCl ratio" indicates

a molar amount of the HCl in the salt formation process based on 1 mol of NBDA; the "phosgene ratio" indicates a molar amount of the phosgene in the phosgenation process based on 1 mol of NBDA hydrochloride; and the "heavy component recovery material ratio" indicates a mass percentage of the heavy component (recovery material) in the mixture in the heavy component recovery process.

Table 3

| | | Example 11 | Example 12 | Example 13 | Example 14 | Example 15 | Comparative example 5 | Comparative example 6 |
|---|---|---|---|---|---|---|---|---|
| Salt formation process | HCl feeding rate (parts by mass per hour) | 75.7 | 94.6 | 113.6 | 132.5 | 189.3 | 189.3 | 75.7 |
| | HCl ratio | 4 | 5 | 6 | 7 | 10 | 10 | 4 |
| Phosgenation process | Phosgene feeding rate (parts by mass per hour) | 513.4 | 513.4 | 641.8 | 641.8 | 1001.2 | 1001.2 | 513.4 |
| | Phosgene ratio | 10 | 10 | 12.5 | 12.5 | 19.5 | 19.5 | 10 |
| | Reaction temperature( °C) | 148 | 149 | 150 | 151 | 152 | 152 | 148 |
| | Reaction pressure (MPa G) | 0.2 | 0.2 | 0.2 | 0.2 | 0.2 | 0.2 | 0.2 |
| | Residence time(h) | 9 | 9 | 9 | 9 | 12 | 12 | 9 |
| Heavy component recovery process | Mixture(parts by mass per hour) | 98.5 | 98.3 | 97.8 | 96.8 | 95.8 | 95.8 | 98.5 |
| | Heavy component recovery material(parts by mass per hour) | 2.0 | 3.4 | 5.6 | 7.4 | 9.6 | 11.5 | 0.5 |
| | Heavy component recovery material ratio (%) | 2 | 3.5 | 5.7 | 7.6 | 10 | 12 | 0.5 |
| Refining process | Withdrawal rate (parts by mass per hour) | 93.1 | 93.2 | 92.6 | 91.6 | 91.0 | 91.0 | 93.1 |
| | Reflux ratio | 10 | 8 | 8 | 6 | 5.5 | 5.5 | 10 |
| NBDI composition | NBDI (%) | >99 | >99 | >99 | >99 | >99 | >99 | >99 |
| | Chlorine (ppm) | 26.9 | 62.1 | 134.9 | 230.9 | 324.6 | 345.1 | 22.9 |
| | CNBI(ppm) | 123 | 220 | 455 | 843 | 1120 | 1120 | 123 |
| | Effective factor E | 5.30 | 4.64 | 4.27 | 4.09 | 3.90 | 3.84 | 6.00 |

Examples 16-18 and Comparative examples 7-9

XDI is prepared using the method described in Example 1 of related art US005196572A, which is taken as Comparative example 7. The Comparative example 7 adopts a thermal cracking process to prepare XDI, where the product contains no chlorine and therefore has no effective factor. The heavy component recovery material of Example 1 is added to the product at a ratio of 4% (i.e., the heavy component in the resulting mixture has a mass percentage of 4%), obtaining Example 16.

Similarly, H6XDI is prepared using the method of Example 1 of US005196572A, which is taken as Comparative example 8, where the raw material is replaced by dimethyl 1,3-cyclohexanedimethylene dicarbamate, with other reaction conditions remaining unchanged. The heavy component recovery material of Example 6 is added to the product at a ratio of 4% (i.e., the heavy component in the resulting mixture has a mass percentage of 4%), obtaining Example 17.

Similarly, NBDI is prepared using the method of Example 1 of US005196572A which is taken as Comparative example 9, where the raw material is replaced by dimethyl norbornanedimethylene dicarbamate. The heavy component recovery material of Example 11 is added to the product at a ratio of 4% (i.e., the heavy component in the resulting mixture has a mass percentage of 4%), obtaining Example 18.

Application example

An optical material, specifically a plastic lens material, is a polythiourethane resin formed by polymerizing isocyanate-based substances and a polythiol compound, where the isocyanate-based substances are isocyanate compositions provided in Examples 1-18 and Comparative examples 1-9, respectively; and the polythiol compound is 1,2-bis((2-mercaptoethyl) thio)-3-mercaptopropane.

The specific preparation method of the optical material is as follows.

0.001 parts by mass of dibutyltin dichloride, 0.07 parts by mass of internal mold release agent (ZELECUN, acidic phosphate, manufactured by Stepan Company), 0.05 part by mass of ultraviolet absorbent (Biosorb 583, manufactured by Sakai Chemical Industry CO., LTD), 36.4 parts by mass of XDI composition (Examples 1 to 5 and 16, Comparative examples 1 to 2 and 7), 37.7 parts by mass of H6XDI composition (Examples 6 to 10 and 17, Comparative examples 3 to 4 and 8), and 39.9 parts by mass of NBDI composition (Examples 11 to 15 and 18, Comparative examples 5 to 6 and 9) were loaded into a flask, followed by stirring at 25 °Cfor 1 h to dissolve them sufficiently, obtaining a polyisocyanate component.

33.6 parts by mass of 1,2-bis((2-mercaptoethyl)thio)-3-mercaptopropane was added to the polyisocyanate component and mixed to prepare a polymerizable composition.

The polymerizable composition was subjected to defoaming treatment at a pressure of 600 Pa for 1 h, filtered through a PTFE filter of 3 um, and then injected into a casting mold formed of a glass mold and an adhesive tape. The casting mold was placed in an oven, and the oven temperature was slowly raised from 10 °C to 120 °C, and polymerization was carried out for 18 h. After polymerization is over, the casting mold was taken out of the oven and demolded to obtain the optical material.

The performance of optical material was tested by the following methods.

(1) Yellowness Index (YI value) test

According to the method in GB/ T 2409-1980, the yellowness index of lens was measured.

The optical material to be tested was made into a circular flat plastic lens with a thickness of 9 mm and a diameter of 75 mm, and tristimulus values x, y and z were determined using a spectrophotometer. The YI value was calculated by the following equation:

$$YI = \frac{100 \times (1.28\ x - 1.06\ z)}{y}.$$

The smaller the YI value, the better the hue of the plastic lens; and the larger the YI value, the worse the hue.

(2) Test of optical deformation occurrence rate

Optical deformation refers to the phenomenon that the local refractive index is different from the surrounding normal refractive index due to the different composition of materials and the like. In the present application, 100 pieces of lenses (which have the same specifications as those of the lenses in the YI value test) were visually observed under a

highpressure mercury lamp. The lenses having striations were determined to be the lens having optical deformation, and the optical deformation occurrence rate was calculated, i.e., the optical deformation occurrence rate =100%× the number of lenses having optical deformation/100.

(3) Test of transmittance

The transmittance of lens was tested according to GB/T 10810.3-2006 "Spectacle lenses and related ophthalmic products-Part 3: Specifications and measurement methods for transmittance".

(4) Test of glass transition temperature (Tg)

The glass transition temperature Tg was measured by differential scanning calorimetry (DSC). Each sample was tested 3 times in parallel and the average value was taken.

Test of glass transition temperature (Tg): high pressure differential scanning calorimeter of METTLER TOLEDO company (METTLER HPDSC1) was used, where DSC test method is as follows: 30 °C to 300 °C, temperature rise scanning, a heating rate of 10 °C/min, nitrogen atmosphere, a nitrogen flow rate of 50 mL/min.

Specific test results are shown in Tables 4, 5 and 6.

Table 4

| | | Example 1 | Example 2 | Example 3 | Example 4 | Example 5 | Example 16 | Compar ative example 1 | Compar ative example 2 | Compar ative example 7 |
|---|---|---|---|---|---|---|---|---|---|---|
| XDI composition | 1,3-XDI (%) | >99 | >99 | >99 | >99 | >99 | >99 | >99 | >99 | >99 |
| | Chlorine (ppm) | 108 | 150 | 230 | 330 | 421.6 | 80 | 440 | 89 | 0 |
| | CBI (ppm) | 450 | 505 | 723 | 1153 | 1517 | 201 | 1517 | 450 | 0 |
| | Effective factor E | 4.70 | 4.29 | 4.05 | 3.98 | 3.90 | 4.39 | 3.84 | 6.00 | -- |
| Optical material | YI value | 1.41 | 1.50 | 1.53 | 1.57 | 1.60 | 1.41 | 1.9 | Gel, untestable | Gel, untestable |
| | Optical deformation occurrence rate (%) | 1.0 | 0.0 | 1.0 | 0.0 | 1.0 | 1.0 | 3.0 | | |
| | Transmittance (%) | 84.10 | 84.07 | 84.02 | 83.98 | 83.95 | 84.10 | 83.80 | | |
| | Tg ( °C) | 85.67 | 85.32 | 85.20 | 84.85 | 84.56 | 85.54 | 83.20 | | |

Table 5

| | | Example 6 | Example 7 | Example 8 | Example 9 | Example 10 | Example 17 | Comparative example 3 | Comparative example 4 | Comparative example 8 |
|---|---|---|---|---|---|---|---|---|---|---|
| H6XDI composition | 1,3-H6XDI(%) | >99 | >99 | >99 | >99 | >99 | >99 | >99 | >99 | >99 |
| | Chlorine (ppm) | 25.6 | 64.7 | 143.9 | 234.3 | 352.5 | 20.4 | 369 | 24.6 | 0 |
| | CIC (ppm) | 125 | 210 | 465 | 853 | 1200 | 53 | 1200 | 125 | 0 |
| | Effective factor E | 5.70 | 4.60 | 4.25 | 4.14 | 3.90 | 4.98 | 3.85 | 6.00 | -- |
| Optical material | YI value | 1.31 | 1.37 | 1.43 | 1.46 | 1.50 | 1.35 | 1.82 | 1.32 | Gel, untestable |
| | Optical deformation occurrence rate (%) | 1 | 0 | 1 | 0 | 1 | 1 | 4 | 5 | |
| | Transmittance (%) | 86.72 | 86.54 | 86.30 | 86.12 | 86.03 | 86.71 | 85.02 | 85.80 | |
| | Tg ( °C) | 89.65 | 89.54 | 89.43 | 89.21 | 89.08 | 89.67 | 88.12 | 88.56 | |

Table 6

| | | Example 11 | Example 12 | Example 13 | Example 14 | Example 15 | Example 15 | Comparative example 5 | Comparative example 6 | Comparative example 9 |
|---|---|---|---|---|---|---|---|---|---|---|
| NBDI composition | NBDI(%) | >99 | >99 | >99 | >99 | >99 | >99 | >99 | >99 | >99 |
| | Chlorine (ppm) | 26.9 | 62.1 | 134.9 | 230.9 | 324.6 | 19 | 345.1 | 22.9 | 0 |
| | CNBI(ppm) | 123 | 220 | 455 | 843 | 1120 | 63 | 1120 | 123 | 0 |
| | Effective factor E | 5.30 | 4.64 | 4.27 | 4.09 | 3.90 | 5.10 | 3.84 | 6.00 | - |
| Optical material | YI value | 1.33 | 1.35 | 1.40 | 1.45 | 1.50 | 1.32 | 1.82 | 1.34 | Gel, untesta ble |
| | Optical deformation occurrence rate (%) | 1 | 0 | 1 | 0 | 1 | 0 | 4 | 5 | |
| | Transmittance (%) | 86.73 | 86.56 | 86.23 | 86.14 | 85.89 | 86.85 | 85.01 | 85.22 | |
| | Tg ( °C) | 108.65 | 108.54 | 108.42 | 108.21 | 108.08 | 108.64 | 107.53 | 107.75 | |

It can be seen from the above performance test data that, in the present application, by controlling the effective factor of the isocyanate composition to be in the range of 3.90 to 5.70, the prepared polythiourethane optical material has lower yellowness index and optical deformation occurrence rate, the optical deformation occurrence rate being 0 to 1%. Where, the yellowness index YI of the optical material prepared with the XDI composition is less than or equal to 1.60, the YI being 1.41 to 1.60, the transmittance is 83.95 % to 84.10 %, and the Tg is 84.56 °C to 85.67 °C; the YI of the optical material prepared with the H6XDI composition is less than or equal to 1.50, the YI being 1.31 to 1.50, the transmittance is 86.03 % to 86.72 %, and the Tg is 89.08 °C to 89.67 °C; and the YI of the optical material prepared with the NBDI composition is less than or equal to 1.50, the YI being 1.32 to 1.50, the transmittance is 85.89 % to 86.85 %, and the Tg is 108.08 °C to 108.65 °C, which also has excellent optical performance and heat resistance.

Applicant states that the isocyanate composition, modified isocyanate, polyurethane resin and optical material of the present application are illustrated by the above examples, but the present application is not limited to the above process steps, that is, it does not mean that the present application must rely on the above process steps to be implemented. Those skilled in the art should understand that any improvement to the present application, equivalent substitution of raw materials selected in the present application, addition of auxiliary components, as well as selection of specific methods and so on all fall within the scope of protection and disclosure of the present application.

## Claims

1. An isocyanate composition, wherein an effective factor of the isocyanate composition is 3.90 to 5.70;

   a calculation formula of the effective factor is shown by Formula I:

$$E = -\lg\left(A - \frac{B \times M_{Cl}}{M_B}\right) \quad \text{Formula I;}$$

   wherein E is an effective factor;
   A is a mass content of chlorine in the isocyanate composition;
   B is a mass content of chloroisocyanate in the isocyanate composition;
   $M_{Cl}$ is relative atomic mass of chlorine; and
   $M_B$ is relative molecular mass of the chloroisocyanate.

2. The isocyanate composition according to claim 1, wherein the A is obtained by X-ray fluorescence testing.

3. The isocyanate composition according to claim 1 or 2, wherein the B is obtained by chromatography-mass spectrometry testing, more preferably, by gas chromatography-mass spectrometry testing.

4. The isocyanate composition according to any one of claims 1 to 3, wherein the isocyanate is diisocyanate, and preferably includes any one of phenylenedimethylene diisocyanate, bis(isocyanatomethyl)cyclohexane and bis(isocyanatomethyl)norborane, or a combination of at least two thereof.

5. The isocyanate composition according to any one of claims 1 to 4, wherein a mass percentage of isocyanate in the isocyanate composition is greater than or equal to 97%.

6. The isocyanate composition according to any one of claims 1 to 5, wherein a substance corresponding to the effective factor comprises any one of the following compounds or a combination of at least two thereof:

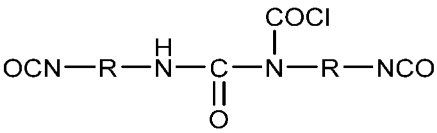
,
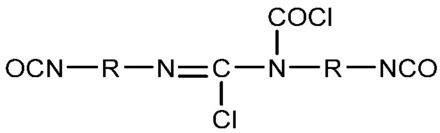
,
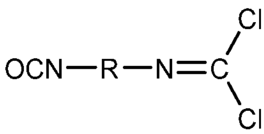
;

wherein R is a divalent group obtained by removing NCO group of isocyanate;
preferably, R is selected from any one of

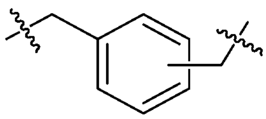
,
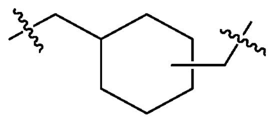
, and

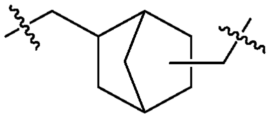

, or a combination of at least two thereof, wherein a wavy line represents a linking site of groups.

7. The isocyanate composition according to any one of claims 1 to 6, wherein the chloroisocyanate is a compound obtained by replacing one NCO group of isocyanate with chlorine;
preferably, the chloroisocyanate comprises any one of

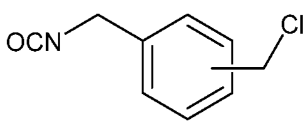
,
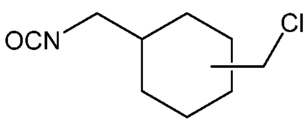
, and
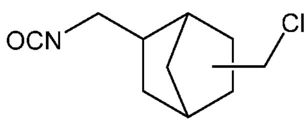
,

or a combination of at least two thereof.

8. A preparation method of the isocyanate composition according to any one of claims 1 to 7, comprising: reacting an amine compound with phosgene to obtain the isocyanate composition.

9. The preparation method according to claim 8, comprising the following steps:

(1) reacting an amine compound with phosgene to obtain a reaction product;
(2) performing a removal treatment on the reaction product obtained in the step (1) to obtain a crude product, wherein the removal treatment comprises phosgene removal treatment and/or solvent removal treatment;
(3) performing separation and refining on the crude product obtained in step (2) to obtain the isocyanate composition.

10. The preparation method according to claim 9, wherein a heavy component and an intermediate product are obtained by the separation in step (3); a mixture of the intermediate product and the heavy component is refined to obtain the isocyanate composition; and a mass percentage of the heavy component in the mixture is 1% to 10%.

11. The preparation method according to claim 10, wherein the refining is a method of rectification.

12. A modified isocyanate, wherein the modified isocyanate is obtained by modifying the isocyanate composition according to any one of claims 1 to 7;
the modified isocyanate comprises any one of groups (a)-(i) or a combination of at least two thereof: (a) isocyanurate group, (b) uretidione group, (c) biuret group, (d) urethane group, (e) urea group, (f) iminooxadiazinedione group, (g) allophanate group, (h) uretonimine group, (i) carbodiimide group.

13. A polyurethane resin, wherein the polyurethane resin is obtained by reacting an isocyanate-based substance with an active hydrogen group-containing substance, and the isocyanate-based substance comprises at least one of the isocyanate composition according to any one of claims 1 to 7 and the modified isocyanate according to claim 12.

14. An optical material, wherein the optical material is obtained by polymerizing an isocyanate-based substance and a polythiol compound, and the isocyanate-based substance comprises at least one of the isocyanate composition according to any one of claims 1 to 7 and the modified isocyanate according to claim 12;
preferably, the polythiol compound comprises at least one of 1,2-bis((2-mercaptoethyl) thio)-3-mercaptopropane, bis(mercaptomethyl)-3,6,9-trithia-1,11-undecanedithiol, pentaerythritol tetra(3-mercaptopropionate), 1,1,3,3-tetra(mercaptomethylthio)propane, or 2-mercaptoethanol.

15. The optical material according to claim 14, wherein the optical material is applied to eyeglasses, lens, prisms, lampshade materials, transparent packaging materials, transparent building materials, or electronic products.

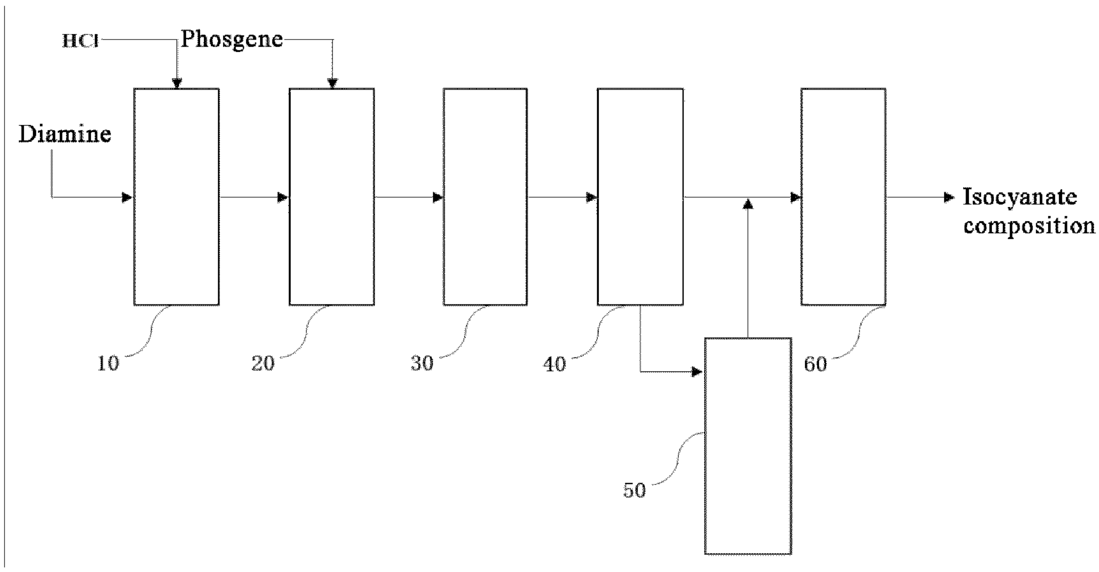
HCl
Phosgene
Diamine
Isocyanate composition
10
20
30
40
50
60

FIG. 1

**INTERNATIONAL SEARCH REPORT**

International application No.
**PCT/CN2022/132417**

**A. CLASSIFICATION OF SUBJECT MATTER**

C07C263/10(2006.01)i; C07C265/14(2006.01)i; C08G18/76(2006.01)i; C08G18/75(2006.01)i; C08G18/38(2006.01)i; C08G18/72(2006.01)i; G02B1/00(2006.01)i; G02B1/04(2006.01)i

According to International Patent Classification (IPC) or to both national classification and IPC

**B. FIELDS SEARCHED**

Minimum documentation searched (classification system followed by classification symbols)

C07C263/-, C07C265/-, C08G18/-, G02B1/-

Documentation searched other than minimum documentation to the extent that such documents are included in the fields searched

Electronic data base consulted during the international search (name of data base and, where practicable, search terms used)

CNTXT, ENTXT, VEN, CNKI: 异氰酸酯, 聚氨酯, 苯二亚甲基二胺, 降冰片烷二甲胺, 环己基二甲胺, 凝胶, 透镜, 白浊, 黄变, xylylene, norbornane, cyclohexyl, diisocyanate, dimethylamine, polyurethane, gel, lens

**C. DOCUMENTS CONSIDERED TO BE RELEVANT**

| Category* | Citation of document, with indication, where appropriate, of the relevant passages | Relevant to claim No. |
|---|---|---|
| X | CN 114031744 A (WANHUA CHEMICAL GROUP CO., LTD.; WANHUA CHEMICAL (NINGBO) CO., LTD.) 11 February 2022 (2022-02-11)<br>description, paragraphs [0219]-[0266], and description, figure 1 | 1-15 |
| X | CN 109153637 A (MITSUI CHEMICALS INC.) 04 January 2019 (2019-01-04)<br>description, paragraphs [0556]-[0626], and description, figure 1 | 1-15 |
| A | CN 112661930 A (WANHUA CHEMICAL GROUP CO., LTD.; WANHUA CHEMICAL (NINGBO) CO., LTD.) 16 April 2021 (2021-04-16)<br>claims 1-7 | 1-15 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 July 2023** | **28 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2022/132417**

| Patent document cited in search report | Publication date (day/month/year) | Patent family member(s) | Publication date (day/month/year) |
|---|---|---|---|
| CN 114031744 A | 11 February 2022 | None | |
| CN 109153637 A | 04 January 2019 | KR 20190129819 A | 20 November 2019 |
| | | US 2019292304 A1 | 26 September 2019 |
| | | US 10640605 B2 | 05 May 2020 |
| | | EP 3825301 A1 | 26 May 2021 |
| | | JP 2018177811 A | 15 November 2018 |
| | | JP 7103879 B2 | 20 July 2022 |
| | | BR 112018074934 A2 | 12 March 2019 |
| | | BR 112018074934 B1 | 28 April 2020 |
| | | EP 3470393 A1 | 17 April 2019 |
| | | EP 3470393 A4 | 11 March 2020 |
| | | EP 3470393 B1 | 03 March 2021 |
| | | WO 2018190290 A1 | 18 October 2018 |
| | | TW 201841880 A | 01 December 2018 |
| | | TWI 655178 B | 01 April 2019 |
| | | KR 20180127517 A | 28 November 2018 |
| | | KR 101970115 B1 | 17 April 2019 |
| CN 112661930 A | 16 April 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101155848 A
- CN 106414538 A
- CN 108084386 A
- US 005196572 A